# EUROPÄISCHE PATENTSCHRIFT

(11) **EP 3 586 124 B1**
(45) Veröffentlichungstag und Bekanntmachung des Hinweises auf die Patenterteilung: **07.04.2021**
(21) Anmeldenummer: 18707630.2
(22) Anmeldetag: 05.02.2018
(51) Int. Cl.: G01N 33/00, G01N 1/40

(54) **SENSORVORRICHTUNG UND VERFAHREN ZUM ERFASSEN VON SUBSTANZEN IN EINEM FLUID**
SENSOR APPARATUS AND METHOD FOR CAPTURING SUBSTANCES IN A FLUID
DISPOSITIF DE DÉTECTION ET PROCÉDÉ PERMETTANT DE DÉTECTER DES SUBSTANCES DANS UN FLUIDE

(30) Priorität: 23.02.2017 DE 102017202918
(43) Veröffentlichungstag der Anmeldung: 01.01.2020
(73) Patentinhaber: Robert Bosch GmbH, 70442 Stuttgart (DE)
(72) Erfinder: BUCK, Thomas, 71732 Tamm (DE); HOFFMANN, Jochen, 71272 Renningen (DE)
(86) Internationale Anmeldenummer: PCT/EP2018/052781
(87) Internationale Veröffentlichungsnummer: WO 2018/153645

(56) Entgegenhaltungen:
- DE-A1- 3 722 379
- JP-A- 2012 181 123
- US-A1- 2003 003 595

## Beschreibung

### Stand der Technik

Die Erfindung geht aus von einer Vorrichtung oder einem Verfahren nach Gattung der unabhängigen Ansprüche.

Für eine Identifikation organischer und/oder anorganischer Verbindungen aus einer Gasphase kann beispielsweise ein definiertes Gasvolumen zunächst in die flüssige Phase überführt und über einen definierten Zeitraum akkumuliert werden, um das Kondensat dann wieder in die Gasphase zu überführen und gezielt auf eine sensierende Oberfläche zu leiten. Herkömmliche Systeme können zusätzlich zu dem eigentlichen Gassensorelement insbesondere Kühleinheiten (z. B. Peltierelemente), mikrofluidische Kanalsysteme (zum gezielten Leiten des Gases), vergrößerte Oberflächen wie z. B. poröse Materialien (um dem vorbei strömenden Gas eine möglichst große Wechselwirkungsoberfläche zu bieten), Heizer (um das Kondensat wieder in die Gasphase zu überführen) aufweisen. Um in Gasen enthaltene Flüssigkeit zu extrahieren, können auch Molekularsiebe, Superadsorber oder chemische Verbindungen wie zum Beispiel Hyaluronsäure verwendet werden.

Aus JP 2012 181123 A ist eine Sensorvorrichtung zum Detektion von Gerüchen bekannt.

### Offenbarung der Erfindung

Vor diesem Hintergrund werden mit dem hier vorgestellten Ansatz eine Sensorvorrichtung gemäß Anspruch 1 und ein Verfahren gemäß Anspruch 8 vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Gemäß Ausführungsformen kann eine integrierte Detektion chemischer Verbindungen bzw. Substanzen mittels der Sensorvorrichtung realisiert werden, die beispielsweise einen Chip mit dem insbesondere piezoelektrisch aktuierbaren Funktionselement aufweist, dessen Temperatur durch ein Kühlelement, z. B. einem Peltier-Element, abgesenkt werden kann. Wenn ein Fluid an einem derartigen Funktionselement vorbeiströmt, kann beispielsweise ein Niederschlag von in einer Gasphase enthaltener Flüssigkeit auf dem Funktionselement induziert werden. Insbesondere durch eine elektrisch induzierte, hochfrequente Schwingung kann anschließend mechanische Energie erzeugt werden, um die kondensierte Flüssigkeit wieder in die Gasphase zu überführen. Das Funktionselement und eine Gassensoroberfläche können hierbei so angeordnet sein, dass gasförmige Substanzen erfasst werden können.

Vorteilhafterweise kann gemäß Ausführungsformen die Sensorvorrichtung mit umfangreicher Funktionalität und auf kleinstem Raum in dem Sensorgehäuse integriert bereitgestellt werden. Weiterhin kann diese Sensorvorrichtung beispielsweise die Möglichkeit bieten, mittels einer geeigneten Auswerteelektronik eine Masse eines Kondensats an dem Funktionselement zu bestimmen. Bei einer Erfassung von hitzelabilen Substanzen bzw. Zielelementen, wie z. B. Proteinen, Humanzellen, Exosomen oder Hormonen, kann anstelle einer thermischen Verdampfung, die zu einer Schädigung von solchen biologischen Substanzen führen könnte, eine Überführung eines Kondensates in die Gasphase bei Raumtemperatur erreicht werden, wodurch eine Schädigung hitzelabiler Substanzen vermieden werden kann. Für eine Überführung eines Kondensats in die Gasphase wird hierbei insbesondere kein Heizelement benötigt. Insbesondere durch eine piezoelektrische Vernebelung oder Zerstäubung kann Energie eingespart werden, was einen im Hinblick auf einen Einsatz in portablen Geräten energiesparenden Betrieb ermöglicht. Beispielsweise können hohe piezoelektrische Verdampfungsraten bzw. Zerstäubungsraten und damit verbundene Geschwindigkeiten bzw. Durchsätze von bis zu 10 Milliliter pro Minute ermöglicht werden. Anders ausgedrückt kann eine Analyse eines großen Gasvolumens bzw. großer Gasvolumina ermöglicht werden. Es können auch Flüssigkeiten analysiert werden. Somit kann ein Einsatzbereich der Sensorvorrichtung weiter vergrößert sein. Ferner kann vermieden werden, dass zwischen einem Kühlen eines Bereichs für die Kondensatbildung und einem Heizen des gleichen Bereiches für das Überführen des Kondensats in die Gasphase umzuschalten ist.

Bei den Substanzen in dem Fluid kann es sich um zumindest einen Stoff, zumindest ein Molekül, zumindest eine chemische Verbindung oder zumindest ein chemisches Element handeln. Die Gassensorvorrichtung kann als ein Sensor-Chip oder dergleichen ausgeführt sein. Die Kühleinrichtung kann ausgebildet sein, um das Funktionselement auf eine Taupunkttemperatur zumindest einer zu erfassenden Substanz zu kühlen.

Das Funktionselement ist als eine perforierte Membran ausgeformt.

Dadurch kann eine Kondensatbildung auf nur einer Seite der Membran erzwungen werden. Wird die Membran nun in mechanische Schwingungen bzw. in Resonanz versetzt, können die generierten Tropfen auf die andere Seite der Membran beschleunigt werden. Dies ermöglicht einen zielgerichteten Transfer auf eine Gassensoroberfläche der Gassensoreinrichtung und kann eine Phasenumwandlung wirksamer machen, was in einer erhöhten Sensitivität der Gassensoreinrichtung resultieren kann.

Auch kann das Funktionselement in dem Gehäuse einseitig eingespannt angeordnet sein. Eine solche Ausführungsform bietet den Vorteil, dass das Funktionselement effizient und exakt in mechanische Schwingungen versetzt werden kann.

Ferner kann das Gehäuse eine erste Kammer und eine zweite Kammer aufweisen. Hierbei können die erste Kammer und die zweite Kammer durch eine Verbindungsöffnung miteinander verbunden sein. Dabei können das Funktionselement, die Schwingungserzeugungseinrichtung und die Kühleinrichtung in der ersten Kammer angeordnet sein. Die Gassensoreinrichtung kann in der zweiten Kammer angeordnet sein. Anders ausgedrückt kann zwischen der ersten Kammer der zweiten Kammer eine Trennwand in dem Gehäuse angeordnet sein. Auf diese Weise kann erreicht werden, dass die zerstäubten Mikrotröpfchen nicht sofort auf eine Sensoroberfläche der Gassensoreinrichtung auftreffen, sondern erst eine gewisse Strecke zurücklegen müssen, in welcher ein Phasenübergang von flüssig zu gasförmig beschleunigt stattfinden kann, bevor sie auf die Sensoroberfläche auftreffen, sodass mehr Bestandteile in die Gasphase übergehen und eine Sensitivität des Sensors verbessert werden kann.

Zudem kann die Sensorvorrichtung ein Kapillarelement aufweisen. Das Kapillarelement kann durch einen Kapillarzwischenraum von dem Funktionselement beabstandet angeordnet sein. Dabei kann der Kapillarzwischenraum angrenzend an die zumindest eine Durchgangsöffnung angeordnet sein. Somit kann eine Flüssigkeit durch Kapillarwirkung von der Durchgangsöffnung in den Kapillarzwischenraum gelangen. Somit können mittels der Sensorvorrichtung auch Flüssigkeiten genau und zuverlässig sowie energiesparend analysiert werden.

Gemäß einer Ausführungsform können in dem Gehäuse zwei Durchgangsöffnungen für einen Durchfluss von Fluid durch das Gehäuse ausgeformt sein. Zusätzlich oder alternativ kann eine Druckausgleichsöffnung zum Druckausgleich zwischen dem Gehäuse und einer Umgebung ausgeformt sein. Es kann ein kontinuierliches und energiesparendes sensieren von Gas oder einer Flüssigkeit ermöglicht werden.

Auch können die Gassensoreinrichtung und die Kühleinrichtung in einem gemeinsamen Substrat und zusätzlich oder alternativ an einem gemeinsamen Substrat ausgeformt sein. Das gemeinsame Substrat kann als ein Chipsubstrat ausgeführt sein. Ferner kann das gemeinsame Substrat eine elektrische Schaltung, insbesondere eine integrierte Schaltung, beispielsweise eine anwendungsspezifische integrierte Schaltung aufweisen. Eine solche Ausführungsform bietet den Vorteil, platzsparend, einfach herstellbar sowie kostengünstig zu sein.

Insbesondere können das Funktionselement, die Schwingungserzeugungseinrichtung und die Kühleinrichtung als ein Stapel angeordnet sein. Auf diese Weise kann das Funktionselement wirksam sowie auf platzsparende Weise gekühlt und in mechanische Schwingungen versetzt werden.

Es wird auch ein Verfahren zum Erfassen von Substanzen in einem Fluid vorgestellt, wobei das Verfahren mit einer Ausführungsform einer hier beschriebenen Sensorvorrichtung ausgeführt wird, wobei das Verfahren zumindest folgende Schritte aufweist:
Ansteuern der Kühleinrichtung, um das Funktionselement zum Kondensieren von Fluid an dem Funktionselement zu kühlen;
Anregen der Schwingungserzeugungseinrichtung, um das Funktionselement zum Zerstäuben von kondensiertem Fluid in mechanische Schwingungen zu versetzen; und
Betreiben der Gassensoreinrichtung, um gasförmige Substanzen in von dem Funktionselement zerstäubtem Fluid zu erfassen.

Im Schritt des Ansteuerns kann ein Ansteuersignal an die Kühleinrichtung angelegt werden. Im Schritt des Anregens kann ein Anregungssignal an die Schwingungserzeugungseinrichtung angelegt werden. Im Schritt des Betreibens kann ein Sensorsignal von der Gassensoreinrichtung empfangen oder eingelesen werden.

Gemäß einer Ausführungsform kann im Schritt des Anregens die Schwingungserzeugungseinrichtung angeregt werden, um in einer ersten Betriebsart zur quantitativen Erfassung das Funktionselement in mechanische Schwingungen mit einer ersten Amplitude und zusätzlich oder alternativ mit einer ersten Frequenz zu versetzen und in einer zweiten Betriebsart zur qualitativen Erfassung das Funktionselement in mechanische Schwingungen mit einer zweiten Amplitude und zusätzlich oder alternativ mit einer zweiten Frequenz zu versetzen. Hierbei kann die erste Amplitude kleiner als die zweite Amplitude sein. Die erste Frequenz und die zweite Frequenz können gleich oder unterschiedlich sein. In der ersten Betriebsart kann somit eine Massenbestimmung des Kondensats an dem Funktionselement ermöglicht werden, wobei ohne konstruktiven Mehraufwand kontinuierlich eine Massenanlagerung an die Kondensatfalle des Sensors gemessen werden kann. Somit können die vom Gassensor erfassten Werte quantitativer erfasst werden, da eine Verbindung zwischen dem analysiertem Luftvolumen und der darin enthaltene Feuchtigkeit hergestellt werden kann.

Auch kann das Verfahren einen Schritt des Förderns eines Fluidstroms in das Gehäuse der Sensorvorrichtung und zusätzlich oder alternativ durch das Gehäuse der Sensorvorrichtung hindurch aufweisen. Eine solche Ausführungsform bietet den Vorteil, dass eine Genauigkeit einer quantitativen Erfassung weiter gesteigert werden kann, da ein definierter Volumenstrom von Fluid zugeführt werden kann.

Ausführungsbeispiele des hier vorgestellten Ansatzes sind in den Zeichnungen dargestellt und in der nachfolgenden Beschreibung näher erläutert. Es zeigt:
Fig. 1 eine schematische Schnittdarstellung einer nicht erfindungsgemäßen Sensorvorrichtung;
Fig. 2 eine schematische Schnittdarstellung einer nicht erfindungsgemäßen Sensorvorrichtung;
Fig. 3 eine schematische Schnittdarstellung einer Sensorvorrichtung gemäß einem Ausführungsbeispiel;
Fig. 4 eine schematische Schnittdarstellung einer Sensorvorrichtung gemäß einem Ausführungsbeispiel;
Fig. 5 eine schematische Schnittdarstellung einer Sensorvorrichtung gemäß einem Ausführungsbeispiel;
Fig. 6 eine schematische Schnittdarstellung einer Sensorvorrichtung gemäß einem Ausführungsbeispiel;
Fig. 7 eine schematische Schnittdarstellung einer Sensorvorrichtung gemäß einem Ausführungsbeispiel; und
Fig. 8 ein Ablaufdiagramm eines Verfahrens zum Erfassen gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

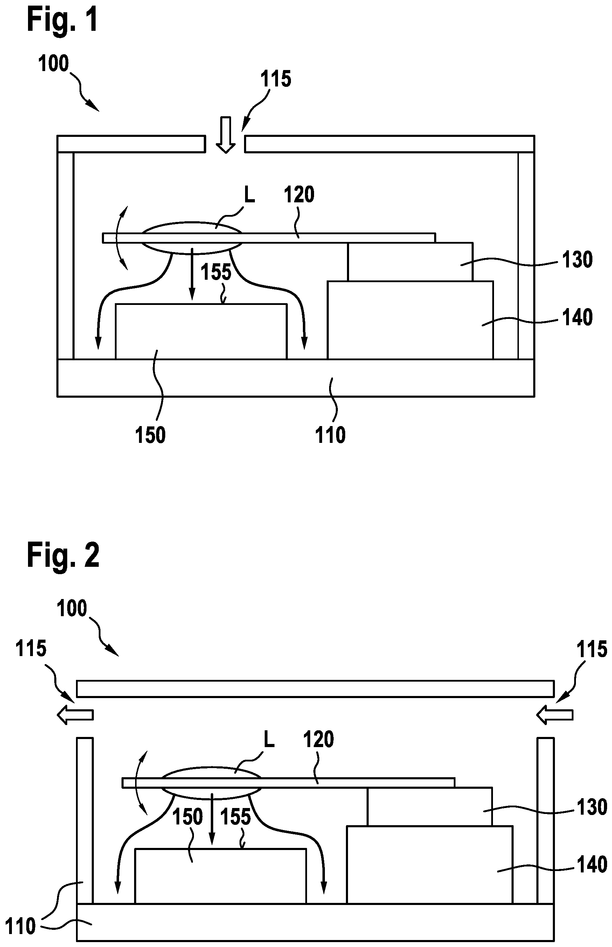

Fig. 1 zeigt eine schematische Schnittdarstellung einer nicht erfindungsgemäßen Sensorvorrichtung 100 zur Erfassung von Substanzen in einem Fluid.

Das Fluid kann zumindest eine Flüssigkeit, zumindest ein Gas oder ein Gas-Flüssigkeit-Gemisch aufweisen. Die zu erfassenden Substanzen können mindestens eine chemische Verbindung, mindestens ein chemisches Element und/oder mindestens ein Molekül repräsentieren.

Die Sensorvorrichtung 100 weist ein Gehäuse 110 auf. In dem Gehäuse 110 ist gemäß dem in Fig. 1 dargestellten Ausführungsbeispiel beispielhaft lediglich eine Durchgangsöffnung 115 für das Fluid ausgeformt. Im Bereich der Durchgangsöffnung 115 ist somit das Gehäuse 110 durchlässig für das Fluid. In dem Gehäuse 110 sind ein Funktionselement 120, eine Schwingungserzeugungseinrichtung 130, eine Kühleinrichtung 140 und eine Gassensoreinrichtung 150 mit einer Gassensoroberfläche 155 angeordnet.

Gemäß dem in Fig. 1 gezeigten Ausführungsbeispiel sind das Funktionselement 120, die Schwingungserzeugungseinrichtung 130 und die Kühleinrichtung 140 als ein Stapel angeordnet bzw. aufeinander gestapelt. Hierbei ist die Schwingungserzeugungseinrichtung 130 zwischen dem Funktionselement 120 und der Kühleinrichtung 140 angeordnet. Die Gassensoreinrichtung 150 ist von dem Stapel beabstandet angeordnet. Dabei ist die Gassensoroberfläche 155 dem Funktionselement 120 bzw. einem Teilabschnitt des Funktionselements 120 zugewandt.

Das Funktionselement 120 ist zum Kondensieren und Zerstäuben von Fluid vorgesehen. Gemäß Fig. 1 ist das Funktionselement 120 als ein Balken ausgeformt. Genau gesagt ist hierbei das Funktionselement 120 als ein einseitig eingespannter Balken ausgeformt bzw. einseitig eingespannt in dem Gehäuse 110 angeordnet. Hierbei ist ein erster Teilabschnitt des Funktionselements 120 an der Schwingungserzeugungseinrichtung 130 angebracht, wobei ein zweiter Teilabschnitt des Funktionselements 120 auskragend ist. Der zweite Teilabschnitt des Funktionselements 120 erstreckt sich über die Gassensoreinrichtung 150 hinweg. Eine Hauptoberfläche des zweiten Teilabschnitts des Funktionselements 120 ist der Gassensoroberfläche 155 der Gassensoreinrichtung 150 zugewandt.

Die Schwingungserzeugungseinrichtung 130 ist ausgebildet, um das Funktionselement 120 in mechanische Schwingungen zu versetzen. Dabei ist die Schwingungserzeugungseinrichtung 130 beispielsweise als ein Ultraschallgenerator, ein piezoelektrisches Element oder dergleichen ausgeführt.

Die Kühleinrichtung 140 ist ausgebildet, um das Funktionselement 120 zu kühlen. Dabei ist die Kühleinrichtung 140 insbesondere ausgebildet, um das Funktionselement 120 auf eine Taupunkttemperatur einer zu erfassenden Substanz in dem Fluid zu kühlen. Die Kühleinrichtung 140 ist beispielsweise als ein Peltier-Element oder dergleichen ausgeführt.

Die Gassensoreinrichtung 150 ist ausgebildet, um gasförmige bzw. wieder in die Gasphase überführte Substanzen in an dem Funktionselement 120 kondensiertem und von dem Funktionselement 120 zerstäubtem Fluid zu erfassen. Dabei ist die Gassensoreinrichtung 150 beispielsweise als ein Gassensorchip mit der Gassensoroberfläche 155 ausgeführt.

Anders ausgedrückt zeigt Fig. 1 einen schematischen Querschnitt durch die Sensorvorrichtung 100. Das Funktionselement 120 in Gestalt eines Balkens ist kraftschlüssig mit der als Ultraschallgenerator bzw. piezoelektrisches Element ausgeführten Schwingungserzeugungseinrichtung 130 verbunden. Die Schwingungserzeugungseinrichtung 130 steht mit einer als Kältegenerator bzw. insbesondere als Peltier-Element ausgeführten Kühleinrichtung 140 in einer thermisch bzw. thermische Energie leitenden Konfiguration bzw. Verbindung. Das Funktionselement 120 ist in unmittelbarer Nähe zu der Gassensoroberfläche 155 der als Gassensorchip ausgeführten Gassensoreinrichtung 150 positioniert. Die vorstehend genannten Elemente sind in dem Gehäuse 110 platziert, das in Gestalt der Durchgangsöffnung 115 eine gasdurchlässige Kontaktstelle zu einem Außenbereich des Gehäuses 110 aufweist.

Um mit einer solchen Sensorvorrichtung 100 in Betrieb Gase zu sensieren, wird die Temperatur des Funktionselements 120 unter Verwendung der Kühleinrichtung 140 mindestens unter den Taupunkt eines zu sensierenden Gases bzw. einer zu erfassenden Substanz abgesenkt. Ein Gasstrom bzw. Fluidstrom kann dem Funktionselement 120 dabei entweder aktiv oder passiv zugeführt werden. Gelöste oder tröpfchenförmige Bestandteile des Gases bzw. Fluids kondensieren an dem gekühlten Funktionselement 120 ab und bilden dort eine wässrige Phase L bzw. ein Kondensat. Die sich an das Funktionselement 120 anlagernden Massen können kontinuierlich durch Messung einer mechanischen Resonanzfrequenz erfasst werden. Dabei ist zu beachten, dass eine Amplitude der Schwingung bei der Messung so eingestellt wird, dass eine unbeabsichtigte Zerstäubung bzw. Vernebelung vermieden wird. Ist beispielsweise eine vordefinierte Masse an wässriger Phase L vorhanden, werden über Wasserstoffbrückenbindungen zusammengehaltene Wassermoleküle durch Einprägen einer großen Bewegung des Funktionselements 120 aus ihrem Molekülverbund gerissen und somit in die Gasphase überführt (Zerstäubung bzw. Vernebelung). Die Arbeitsfrequenz beim Vernebeln braucht nicht der Messfrequenz bei der Massenbestimmung zu entsprechen. Es kann vorteilhaft sein, beim Vernebeln eine höhere Schwingungsmode anzuregen. Gemäß dem in Fig. 1 gezeigten Ausführungsbeispiel sind das Funktionselement 120 und die Gassensoroberfläche 155 einander direkt gegenüberliegend angeordnet.

Fig. 2 zeigt eine schematische Schnittdarstellung einer nicht erfindungsgemäßen Sensorvorrichtung 100. Diese entspricht der der Sensorvorrichtung aus Fig. 1 mit Ausnahme dessen, dass in dem Gehäuse 110 zwei Durchgangsöffnungen 115 für einen Durchfluss von Fluid durch das Gehäuse 110 ausgeformt sind.

Somit weist das Gehäuse 110 mit den Durchgangsöffnungen 115 mindestens zwei gasdurchlässige Kontaktstellen zum Außenbereich des Gehäuses 110 auf. Dies hat den Vorteil, dass definierte Gasvolumina unter Verwendung eines Gasfördersystems, z. B. einer Mikropumpe, durch das Gehäuse 110 und insbesondere über das Funktionselement 120 geleitet werden können. Dies kann eine Aussagekraft von Messungen erhöhen, da ein Zusammenhang zwischen analysiertem Gasvolumen und Messsignalen hergestellt werden kann.

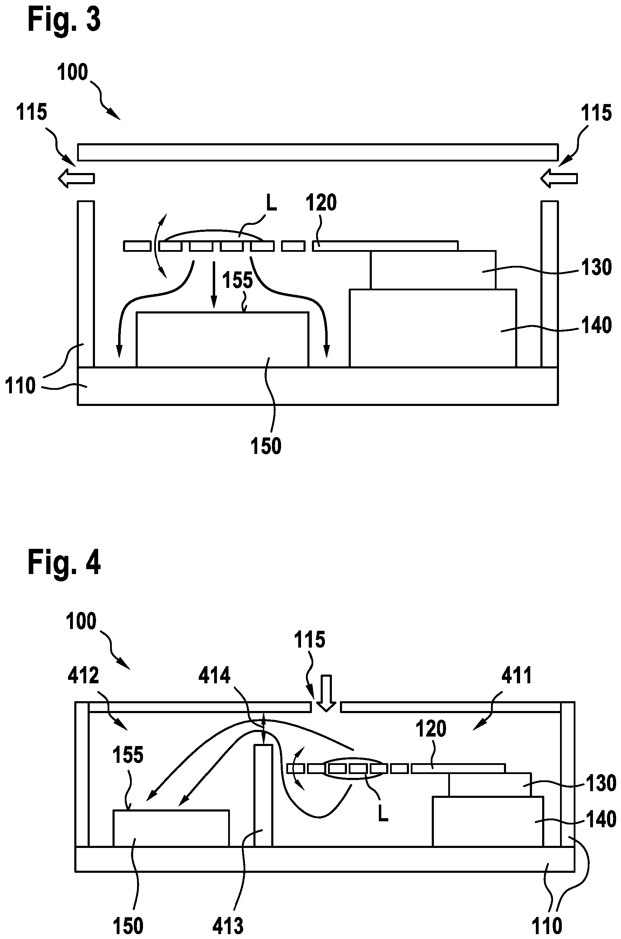

Fig. 3 zeigt eine schematische Schnittdarstellung einer Sensorvorrichtung 100 gemäß einem Ausführungsbeispiel. Die Sensorvorrichtung 100 ähnelt oder entspricht der Sensorvorrichtung aus einer der vorstehend beschriebenen Figuren. Dabei entspricht die Sensorvorrichtung 100 der Sensorvorrichtung aus Fig. 2 mit Ausnahme dessen, dass das Funktionselement 120 als eine Membran ausgeformt ist. Erfindungsgemäß ist die Membran perforiert.

Das als Membran ausgeführte Funktionselement 120 hat in Kombination mit der Durchflusskonfiguration aus Fig. 2 den Vorteil, dass die Kondensatbildung bzw. Anlagerung der flüssigen Phase L beispielsweise auf nur einer Seite der Membran erzwungen werden kann. Wird das Funktionselement 120 nun in Resonanz versetzt, werden die generierten Tropfen auf die andere Seite des Funktionselements 120 beschleunigt. Dies ermöglicht einen zielgerichteten Transfer auf die Gassensoroberfläche 155 und reduziert Verluste bei der Phasenumwandlung, was in einer erhöhten Sensitivität der Sensorvorrichtung 100 resultiert.

Fig. 4 zeigt eine schematische Schnittdarstellung einer Sensorvorrichtung 100 gemäß einem Ausführungsbeispiel. Die Sensorvorrichtung 100 ähnelt oder entspricht der Sensorvorrichtung aus Fig. 3 mit Ausnahme dessen, dass lediglich eine Durchgangsöffnung 115 in dem Gehäuse 110 ausgeformt ist und das Gehäuse 110 eine erste Kammer 411 und eine zweite Kammer 412 aufweist.

Die erste Kammer 411 und die zweite Kammer 412 sind durch eine Trennwand 413 partiell voneinander getrennt und über eine Verbindungsöffnung 414 bzw. einen Verbindungsspalt 414 miteinander verbunden. Der Verbindungsspalt 414 erstreckt sich zwischen der Trennwand 413 und einer Wand des Gehäuses 110. Das Funktionselement 120, die Schwingungserzeugungseinrichtung 130 und die Kühleinrichtung 140 sind in der ersten Kammer 411 angeordnet. Die Gassensoreinrichtung 150 ist in der zweiten Kammer 412 angeordnet. Somit ist die Trennwand 413 zwischen der Gassensoreinrichtung 150 und dem Stapel aus Funktionselement 120, Schwingungserzeugungseinrichtung 130 und Kühleinrichtung 140 angeordnet.

Beide Kammern 411 und 412 bzw. beide Kompartimente sind durch eine den Gasaustausch begünstigende bzw. ermöglichende Öffnung in Gestalt des Verbindungsspalts 414 verbunden. Dieses Ausführungsbeispiel hat den Vorteil, dass die mittels der Schwingungserzeugungseinrichtung 130 generierten Mikrotröpfchen nicht sofort auf die Sensoroberfläche 155 auftreffen, sondern erst eine gewisse Strecke zurücklegen, in welcher der Phasenübergang von flüssig zu gasförmig aufgrund eines vergrößerten Oberflächen-zu-Volumen-Verhältnisses beschleunigt stattfinden kann, bevor sie auf die Sensoroberfläche 155 auftreffen. Dadurch gehen insbesondere mehr Bestandteile in die Gasphase über, was in einer verbesserten Sensitivität der Sensorvorrichtung 100 resultiert.

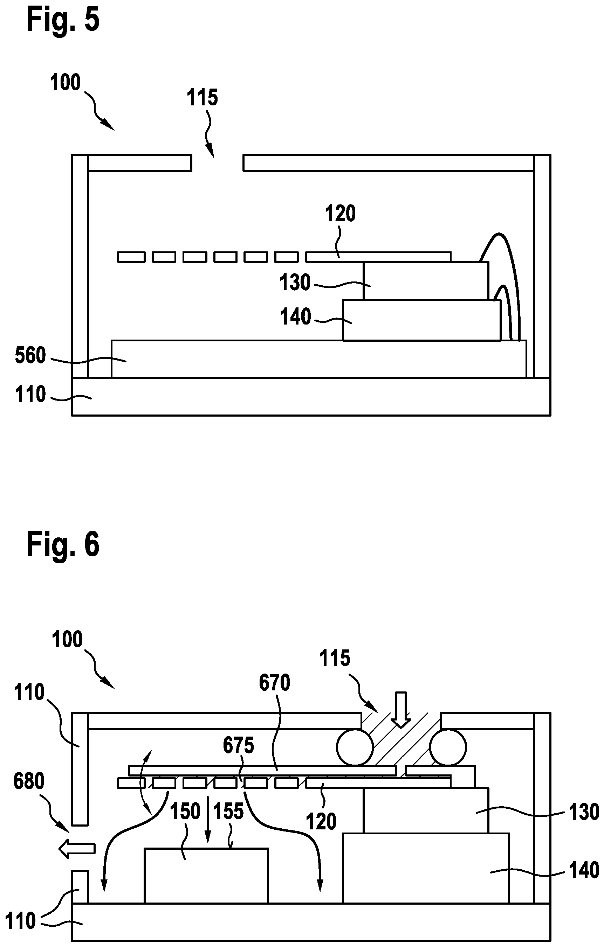

Fig. 5 zeigt eine schematische Schnittdarstellung einer Sensorvorrichtung 100 gemäß einem Ausführungsbeispiel. Die Sensorvorrichtung 100 ähnelt oder entspricht der Sensorvorrichtung aus Fig. 3 mit Ausnahme dessen, dass in dem Gehäuse 110 lediglich eine Durchgangsöffnung 115 ausgeformt ist und die Gassensoreinrichtung und die Kühleinrichtung 140 in bzw. an einem gemeinsamen Substrat 560 bzw. Chipsubstrat 560 ausgeformt bzw. aufgebaut sind.

Anders ausgedrückt sind der Gassensorchip bzw. die Gassensoreinrichtung und die Kühleinrichtung 140 monolithisch auf dem Chipsubstrat 560 aufgebaut. In dem Chipsubstrat 560 kann zusätzlich noch eine anwendungsspezifische integrierte Schaltung (ASIC = Application-Specific Integrated Circuit) für die Datenverarbeitung ausgeformt bzw. angeordnet sein. Dies hat den Vorteil einer kleineren Gesamtbaugröße, was im Hinblick auf den Einsatz in portablen Geräten vorteilhaft ist. Zusätzlich vereinfacht sich eine Aufbau- und Verbindungstechnik durch weniger Prozessschritte, was wiederum Gesamtkosten der Sensorvorrichtung 100 reduziert.

Fig. 6 zeigt eine schematische Schnittdarstellung einer Sensorvorrichtung 100 gemäß einem Ausführungsbeispiel. Die Sensorvorrichtung 100 ähnelt oder entspricht der Sensorvorrichtung aus Fig. 3 mit Ausnahme dessen, dass das Gehäuse 110 lediglich eine Durchgangsöffnung 115 aufweist, die Sensorvorrichtung 100 ein Kapillarelement 670 aufweist, dass durch einen Kapillarzwischenraum 675 bzw. kapillaren Spalt 675 von dem Funktionselement 120 beabstandet angeordnet ist, und in dem Gehäuse 110 eine Druckausgleichsöffnung 680 zum Druckausgleich zwischen dem Gehäuse 110 und einer Umgebung ausgeformt ist. Dabei ist der Kapillarzwischenraum 675 angrenzend an die Durchgangsöffnung 115 angeordnet.

Hierbei gelangt das fluide in Gestalt einer zu analysierenden Flüssigkeit über die Durchgangsöffnung 115 in Kontakt mit dem kapillaren Spalt 675, wodurch die Flüssigkeit in das Gehäuse 110 bzw. Sensorgehäuse transportiert wird. Der kapillare Spalt 675 ist durch eine dem als Membran ausgeführten Funktionselement 120 gegenüberliegende Schicht als Kapillarelement 670 in Zusammenwirkung mit dem Funktionselement 120 realisiert. Wird das Funktionselement 120 in Schwingung versetzt, werden die erzeugten Tröpfchen gerichtet in Richtung der Sensoroberfläche 155 beschleunigt und in die Gasphase übergegangene Stoffe können von der Sensoroberfläche 155 erfasst werden. Gemäß diesem Ausführungsbeispiel weist das Gehäuse 110 eine zusätzliche Öffnung als Druckausgleichsöffnung 680 auf, um einen Druckausgleich zwischen Innengehäuse bzw. Gehäuseinnenraum und der Umgebung herzustellen, wodurch die erfassten Gase und generierte Luftfeuchtigkeit wieder aus dem Gehäuse 110 herausgeführt werden können.

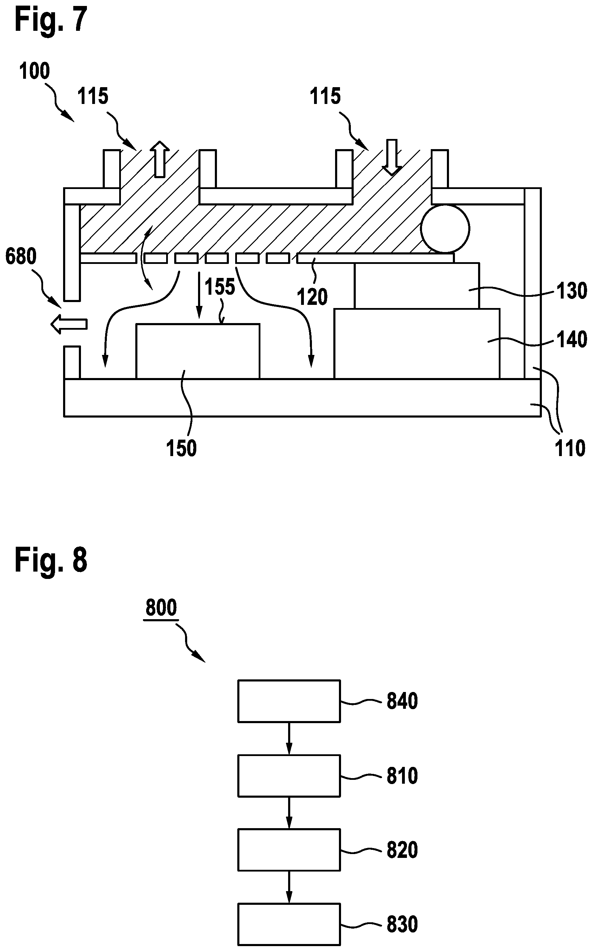

Fig. 7 zeigt eine schematische Schnittdarstellung einer Sensorvorrichtung 100 gemäß einem Ausführungsbeispiel. Die Sensorvorrichtung 100 ähnelt oder entspricht der Sensorvorrichtung aus einer der vorstehend beschriebenen Figuren. Dabei entspricht die Sensorvorrichtung 100 der Sensorvorrichtung aus Fig. 3 mit Ausnahme dessen, dass das als Membran ausgeformte Funktionselement 120 mehr als einseitig eingespannt ist und einen Bereich der zwei Durchgangsöffnungen 115 von einem die Gassensoreinrichtung 150 aufweisenden Innenraum des Gehäuses 110 abtrennt, und dass in dem Gehäuse 110 eine Druckausgleichsöffnung 680 zum Druckausgleich zwischen dem Gehäuse 110 und einer Umgebung ausgeformt ist. Hierbei erstreckt sich das Funktionselement 120 zwischen den Durchgangsöffnungen 115 auf einer Seite und der Druckausgleichsöffnung 680 sowie der Gassensoreinrichtung 150 auf der anderen Seite.

Anders ausgedrückt weist das Gehäuse 110 die beiden Durchgangsöffnungen 115 als Einlass und Auslass auf, um Flüssigkeiten in definierten Durchflussraten über das als Membran ausgeführte Funktionselement 120 zu leiten. Wird das Fusionselement 120 in Schwingung versetzt, werden dem Flüssigkeitsstrom kontinuierlich Teilvolumina entnommen und der Gassensoroberfläche 155 zugeführt. Dies hat den Vorteil, dass Flüssigkeiten kontinuierlich analysiert und/oder überwacht werden können.

Fig. 8 zeigt ein Ablaufdiagramm eines Verfahrens 800 zum Erfassen zum Erfassen von Substanzen in einem Fluid, unter Verwendung der Sensorvorrichtung 100 aus einer der vorstehend genannten Figuren 3-7.

In einem Schritt 810 des Ansteuerns wird bei dem Verfahren 800 zum Erfassen die Kühleinrichtung der Sensorvorrichtung angesteuert, um das Funktionselement zum Kondensieren von Fluid an dem Funktionselement zu kühlen. Hierbei wird beispielsweise ein Ansteuersignal verwendet. Das Funktionselement wird insbesondere auf eine Taupunkttemperatur zumindest einer Substanz in dem Fluid gekühlt. Nachfolgend wird in einem Schritt 820 des Anregens die Schwingungserzeugungseinrichtung der Sensorvorrichtung angeregt, um das Funktionselement in mechanische Schwingungen zu versetzen, um kondensiertes Fluid zu zerstäuben. Dabei wird beispielsweise ein Anregungssignal verwendet. Nachfolgend wird in einem Schritt 830 des Betreibens die Gassensoreinrichtung betrieben bzw. ein Betrieb derselben gesteuert, um gasförmige Substanzen in von dem Funktionselement zerstäubtem Fluid zu erfassen. Hierbei wird insbesondere ein Sensorsignal von der Gassensoreinrichtung empfangen, eingelesen und/oder ausgewertet.

Gemäß einem Ausführungsbeispiel weist das Verfahren 800 zum Erfassen auch einen Schritt 840 des Förderns eines Fluidstroms in das Gehäuse der Sensorvorrichtung und/oder durch das Gehäuse der Sensorvorrichtung hindurch auf. Dabei ist der Schritt 840 des Förderns kontinuierlich ausführbar. Anders ausgedrückt sind der Schritt 810 des Ansteuerns, der Schritt 820 des Anregens und der Schritt 830 des Betreibens während einer Ausführung des Schrittes 840 des Förderns ausführbar.

Gemäß einem Ausführungsbeispiel wird im Schritt 820 des Anregens die Schwingungserzeugungseinrichtung angeregt, um in einer ersten Betriebsart zur quantitativen Erfassung das Funktionselement in mechanische Schwingungen mit einer ersten Amplitude und/oder mit einer ersten Frequenz zu versetzen und in einer zweiten Betriebsart zur qualitativen Erfassung das Funktionselement in mechanische Schwingungen mit einer zweiten Amplitude und/oder mit einer zweiten Frequenz zu versetzen. Die erste Amplitude ist dabei kleiner als die zweite Amplitude. Die erste Frequenz und die zweite Frequenz sind gleich oder unterschiedlich.

Unter Bezugnahme auf die vorstehend beschriebenen Figuren werden nachfolgend exemplarische und spezifische Einzelheiten und/oder Parameter von Ausführungsbeispielen näher erläutert.

Die Kühleinrichtung 140 ist beispielsweise als ein Peltier-Element ausgeführt. Peltier-Elemente sind insbesondere auch in kleinen Abmessungen verfügbar. Für einen Einbau in das Gehäuse 110 kann ein solches Element beispielsweise eine Standfläche kleiner oder gleich 3 mal 3 Quadratmillimeter aufweisen. Optional kann eine Passivierung vorgesehen sein, um das Peltier-Element bzw. die Kühleinrichtung 140 vor Umwelteinflüssen, insbesondere Feuchtigkeit, zu schützen. Durch eine solche Passivierung kann auch verhindert werden, dass Materialien des Peltier-Elements, wie beispielsweise Wismut, Tellur und dergleichen, sich durch Ausdiffundieren auf die Sensorfunktion auswirken können. Als Passivierung können organische Materialien, z. B. Parylene, oder anorganische Materialien, wie beispielsweise Aluminiumoxid, Siliziumoxid etc., verwendet werden.

Für die Schwingungserzeugungseinrichtung 130 bzw. den Ultraschallgenerator können beispielsweise Piezoelectric Micromachined Ultrasonic Transducers (cMUTs, pMUTs), piezoelektrische Folien (PVDF = Polyvinylidenfluorid), piezoelektrisch Kristalle, wie z. B. Blei-Zirkonat-Titanat (PZT), Quarz, Lithiumniobat, Galliumorthophosphat, Ferroelektrika, oder SAW-Systeme (SAW = Surface Acoustic Wave, akustische Oberflächenwelle; typischerweise basierend auf Aluminiumnitrid (AIN)). Es kann vorteilhaft sein, das piezoelektrische Element bzw. die Schwingungserzeugungseinrichtung 130 ebenfalls durch eine Passivierungsschicht vor Umwelteinflüssen und einem Ausdiffundieren zu schützen.

In einem besonders günstigen Ausführungsbeispiel kann eine Länge des Funktionselementes 120 1mm betragen, wobei 0,7mm davon freigestellt sind. Die Breite des Funktionselementes 120 kann beispielsweise 0,5mm und die Dicke des Funktionselementes 120 20 µm betragen. Die Durchmesser der Löcher im Funktionselement 120 können gemäß einem Ausführungsbeispiel 20 µm und/oder deren Abstand zueinander beispielsweise 100 µm betragen, wobei die Löcher beispielsweise matrixförmig angeordnet sind. Die Piezo-Schicht bzw. Schwingungserzeugungseinrichtung 130 ist beispielsweise 5 µm dick, wobei der Sockel (der auf dem Peltier-Element sitzen würde) bzw. die Kühleinrichtung 140 beispielsweise 0,3 mm hoch und 0,3 mm breit sein können.

Das Funktionselement 120 kann beispielsweise aus Stahl, Stahl mit vergüteter Oberfläche, z. B. Silber, Gold, Nickel oder ein anderes Metall, das korrosionsfrei und ggf. lötbar ist, etc. ausgeformt sein. Die Schwingungserzeugungseinrichtung 130 kann beispielsweise aus Blei-Zirkonat-Titanat ausgeformt sein. Ein Substrat kann beispielsweise aus Silizium oder Stahl ausgeformt sein oder direkt Teil der Kühleinrichtung 140 bzw. eines Peltier-Kühl-/Heizelements sein.

Damit ergibt sich eine Resonanzfrequenz der mechanischen Schwingungen des Funktionselements von beispielsweise ca. 37 kHz, also außerhalb des akustisch hörbaren Bereichs. Ein nächstfolgender Modus der Resonanzschwingung würde bei 207 kHz liegen. Zum Messen der angelagerten Masse über die

Frequenzverschiebung können beide hier genannten Modi verwendet werden, wobei der erste Modus eine höhere Empfindlichkeit zeigen wird.

Aufgebaut wird die Schwingungserzeugungseinrichtung 130 insbesondere durch Aufkleben oder Auflöten eines dünnen Stahlblechs auf einen Rahmen bzw. Sockel. Eine Piezo-Schicht kann optional vorher auflaminiert und/oder aufgelötet werden. Das Funktionselement 120 sowie Löcher werden beispielsweise durch Laser strukturiert, insbesondere im Vorfeld.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Sensorvorrichtung (100) zum Erfassen von Substanzen in einem Fluid, wobei die Sensorvorrichtung (100) zumindest folgende Merkmale aufweist:
ein Funktionselement (120) zum Kondensieren und Zerstäuben von Fluid;
eine Schwingungserzeugungseinrichtung (130) zum Versetzen des Funktionselements (120) in mechanische Schwingungen;
eine Kühleinrichtung (140) zum Kühlen des Funktionselements (120);
eine Gassensoreinrichtung (150) zum Erfassen von gasförmigen Substanzen in von dem Funktionselement (120) zerstäubtem Fluid; und
ein Gehäuse (110) zum Aufnehmen des Funktionselements (120), der Schwingungserzeugungseinrichtung (130), der Kühleinrichtung (140) und der Gassensoreinrichtung (150), wobei in dem Gehäuse (110) zumindest eine Durchgangsöffnung (115) für das Fluid ausgeformt ist, **dadurch gekennzeichnet, dass** das Funktionselement (120) als eine perforierte Membran ausgeformt ist.

2. Sensorvorrichtung (100) gemäß einem der vorangegangenen Ansprüche, **dadurch gekennzeichnet, dass** das Funktionselement (120) in dem Gehäuse (110) einseitig eingespannt angeordnet ist.

3. Sensorvorrichtung (100) gemäß einem der vorangegangenen Ansprüche, **dadurch gekennzeichnet, dass** das Gehäuse (110) eine erste Kammer (411) und eine zweite Kammer (412) aufweist, wobei die erste Kammer (411) und die zweite Kammer (412) durch eine Verbindungsöffnung (414) miteinander verbunden sind, wobei das Funktionselement (120), die Schwingungserzeugungseinrichtung (130) und die Kühleinrichtung (140) in der ersten Kammer (411) angeordnet sind, wobei die Gassensoreinrichtung (150) in der zweiten Kammer (412) angeordnet ist.

4. Sensorvorrichtung (100) gemäß einem der vorangegangenen Ansprüche, **gekennzeichnet durch** ein Kapillarelement (670), wobei das Kapillarelement (670) durch einen Kapillarzwischenraum (675) von dem Funktionselement (120) beabstandet angeordnet ist, wobei der Kapillarzwischenraum (675) angrenzend an die zumindest eine Durchgangsöffnung (115) angeordnet ist.

5. Sensorvorrichtung (100) gemäß einem der vorangegangenen Ansprüche, **dadurch gekennzeichnet, dass** in dem Gehäuse (110) zwei Durchgangsöffnungen (115) für einen Durchfluss von Fluid durch das Gehäuse (110) ausgeformt sind und/oder eine Druckausgleichsöffnung (680) zum Druckausgleich zwischen dem Gehäuse (110) und einer Umgebung ausgeformt ist.

6. Sensorvorrichtung (100) gemäß einem der vorangegangenen Ansprüche, **dadurch gekennzeichnet, dass** die Gassensoreinrichtung (150) und die Kühleinrichtung (140) in und/oder an einem gemeinsamen Substrat (560) ausgeformt sind.

7. Sensorvorrichtung (100) gemäß einem der vorangegangenen Ansprüche, **dadurch gekennzeichnet, dass** das Funktionselement (120), die Schwingungserzeugungseinrichtung (130) und die Kühleinrichtung (140) als ein Stapel angeordnet sind.

8. Verfahren (800) zum Erfassen von Substanzen in einem Fluid, wobei das Verfahren mit einer Sensorvorrichtung (100) gemäß einem der vorangegangenen Ansprüche ausgeführt wird, wobei das Verfahren (800) zumindest folgende Schritte aufweist:
Ansteuern (810) der Kühleinrichtung (140), um das Funktionselement (120) zum Kondensieren von Fluid an dem Funktionselement zu kühlen;
Anregen (820) der Schwingungserzeugungseinrichtung (130), um das Funktionselement (120) zum Zerstäuben von kondensiertem Fluid in mechanische Schwingungen zu versetzen; und
Betreiben (830) der Gassensoreinrichtung, um gasförmige Substanzen in von dem Funktionselement (120) zerstäubtem Fluid zu erfassen.

9. Verfahren (800) gemäß Anspruch 8, **dadurch gekennzeichnet, dass** im Schritt (820) des Anregens die Schwingungserzeugungseinrichtung (130) angeregt wird, um in einer ersten Betriebsart zur quantitativen Erfassung das Funktionselement (120) in mechanische Schwingungen mit einer ersten Amplitude und/oder mit einer ersten Frequenz zu versetzen und in einer zweiten Betriebsart zur qualitativen Erfassung das Funktionselement (120) in mechanische Schwingungen mit einer zweiten Amplitude und/oder mit einer zweiten Frequenz zu versetzen, wobei die erste Amplitude kleiner als die zweite Amplitude ist, wobei die erste Frequenz und die zweite Frequenz gleich oder unterschiedlich sind.

10. Verfahren (800) gemäß einem der Ansprüche 8 bis 9, **gekennzeichnet durch** einen Schritt (840) des Förderns eines Fluidstroms in das Gehäuse (110) der Sensorvorrichtung (100) und/oder durch das Gehäuse (110) der Sensorvorrichtung (100) hindurch.

## Claims

1. Sensor device (100) for detecting substances in a fluid, wherein the sensor device (100) has at least the following features:
a functional element (120) for condensing and atomizing fluid;
an oscillation generator (130) for setting the functional element (120) into mechanical oscillations;
a cooling apparatus (140) for cooling the functional element (120);
a gas sensor apparatus (150) for detecting gaseous substances in fluid atomized by the functional element (120); and
a housing (110) for receiving the functional element (120), the oscillation generator (130), the cooling apparatus (140) and the gas sensor apparatus (150), wherein at least one through-aperture (115) for the fluid is formed in the housing (110), **characterized in that** the functional element (120) is formed as a perforated membrane.

2. Sensor device (100) according to one of the preceding claims, **characterized in that** the functional element (120) is arranged so as to be clamped in the housing (110) on one side.

3. Sensor device (100) according to one of the preceding claims, **characterized in that** the housing (110) has a first chamber (411) and a second chamber (412), wherein the first chamber (411) and the second chamber (412) are connected to one another by a connecting aperture (414), wherein the functional element (120), the oscillation generator (130) and the cooling apparatus (140) are arranged in the first chamber (411), wherein the gas sensor apparatus (150) is arranged in the second chamber (412) .

4. Sensor device (100) according to one of the preceding claims, **characterized by** a capillary element (670), wherein the capillary element (670) is arranged spaced apart from the functional element (120) by a capillary intermediate space (675), wherein the capillary intermediate space (675) is arranged adjacent to the at least one through-aperture (115).

5. Sensor device (100) according to one of the preceding claims, **characterized in that** two through-apertures (115) for a flow of fluid through the housing (110) are formed in the housing (110) and/or a pressure equalization aperture (680) is formed for pressure equalization between the housing (110) and an environment.

6. Sensor device (100) according to one of the preceding claims, **characterized in that** the gas sensor apparatus (150) and the cooling apparatus (140) are formed in and/or on a common substrate (560).

7. Sensor device (100) according to one of the preceding claims, **characterized in that** the functional element (120), the oscillation generator (130) and the cooling apparatus (140) are arranged in the form of a stack.

8. Method (800) for detecting substances in a fluid, wherein the method is executed in connection with a sensor device (100) according to one of the preceding claims, wherein the method (800) has at least the following steps:
actuating (810) the cooling apparatus (140) so as to cool the functional element (120) in order to condense fluid on the functional element;
exciting (820) the oscillation generator (130) so as to set the functional element (120) into mechanical oscillations in order to atomize condensed fluid; and
operating (830) the gas sensor apparatus so as to detect gaseous substances in fluid atomized by the functional element (120).

9. Method (800) according to Claim 8, **characterized in that**, in the excitation step (820), the oscillation generator (130) is excited so as, in a first quantitative detection mode of operation, to set the functional element (120) into mechanical oscillations at a first amplitude and/or at a first frequency and so as, in a second qualitative detection mode of operation, to set the functional element (120) into mechanical oscillations at a second amplitude and/or at a second frequency, wherein the first amplitude is less than the second amplitude, wherein the first frequency and the second frequency are the same or different.

10. Method (800) according to either of Claims 8 and 9, **characterized by** a step (840) of conveying a fluid flow into the housing (110) of the sensor device (100) and/or through the housing (110) of the sensor device (100).

## Revendications

1. Dispositif de détection (100) destiné à détecter des substances dans un fluide, le dispositif de détection (100) présentant au moins les caractéristiques suivantes :
un élément fonctionnel (120) destiné à condenser et atomiser un fluide ;
un dispositif de génération de vibrations (130) destiné à faire vibrer l'élément fonctionnel (120) mécaniquement ;
un dispositif de refroidissement (140) destiné à refroidir l'élément fonctionnel (120) ;
un dispositif de détection de gaz (150) destiné à détecter des substances gazeuses dans un fluide atomisé par l'élément fonctionnel (120) ; et
un boîtier (110) destiné à recevoir l'élément fonctionnel (120), le dispositif de génération de vibrations (130), le dispositif de refroidissement (140) et le dispositif de détection de gaz (150), au moins une ouverture de passage (115) destinée au fluide étant ménagée dans le boîtier (110), **caractérisé en ce que** l'élément fonctionnel (120) est réalisé sous la forme d'une membrane perforée.

2. Dispositif de détection (100) selon l'une des revendications précédentes, **caractérisé en ce que** l'élément fonctionnel (120) est disposé dans le boîtier (110) en étant serré d'un côté.

3. Dispositif de détection (100) selon l'une des revendications précédentes, **caractérisé en ce que** le boîtier (110) comporte une première chambre (411) et une deuxième chambre (412), la première chambre (411) et la deuxième chambre (412) étant reliées l'une à l'autre par une ouverture de liaison (414), l'élément fonctionnel (120), le dispositif de génération de vibrations (130) et le dispositif de refroidissement (140) étant disposés dans la première chambre (411), le dispositif de détection de gaz (150) étant disposé dans la deuxième chambre (412).

4. Dispositif de détection (100) selon l'une des revendications précédentes, **caractérisé par** un élément capillaire (670), l'élément capillaire (670) étant disposé à distance de l'élément fonctionnel (120) par le biais d'un espace intermédiaire capillaire (675), l'espace intermédiaire capillaire (675) étant disposé de manière adjacente à l'au moins une ouverture de passage (115).

5. Dispositif de détection (100) selon l'une des revendications précédentes, **caractérisé en ce que** deux ouvertures de passage (115) destinées à un écoulement de fluide à travers le boîtier (110) sont ménagées dans le boîtier (110) et/ou une ouverture de compensation de pression (680) est ménagée pour compenser la pression entre le boîtier (110) et un environnement.

6. Dispositif de détection (100) selon l'une des revendications précédentes, **caractérisé en ce que** le dispositif de détection de gaz (150) et le dispositif de refroidissement (140) sont formés dans et/ou sur un substrat commun (560).

7. Dispositif de détection (100) selon l'une des revendications précédentes, **caractérisé en ce que** l'élément fonctionnel (120), le dispositif de génération de vibrations (130) et le dispositif de refroidissement (140) sont disposés sous la forme d'un empilement.

8. Procédé (800) de détection de substances dans un fluide, le procédé étant mis en œuvre au moyen d'un dispositif de détection (100) selon l'une des revendications précédentes, le procédé (800) comprenant au moins les étapes suivantes :
commander (810) le dispositif de refroidissement (140) afin de refroidir l'élément fonctionnel (120) pour condenser le fluide sur l'élément fonctionnel ;
exciter (820) le dispositif de génération de vibrations (130) afin de faire vibrer l'élément fonctionnel (120) mécaniquement pour atomiser le fluide condensé ; et
faire fonctionner (830) le dispositif de détection de gaz pour détecter des substances gazeuses dans le fluide atomisé par l'élément fonctionnel (120).

9. Procédé (800) selon la revendication 8, **caractérisé en ce que**, à l'étape (820) d'excitation, le dispositif de génération de vibrations (130) est excité afin de faire vibrer mécaniquement l'élément fonctionnel (120) à une première amplitude et/ou à une première fréquence dans un premier mode de fonctionnement destiné à la détection quantitative, et pour faire vibrer mécaniquement l'élément fonctionnel (120) à une deuxième amplitude et/ou à une deuxième fréquence dans un deuxième mode de fonctionnement destiné à la détection qualitative, la première amplitude étant inférieure à la deuxième amplitude, la première fréquence et la deuxième fréquence étant identiques ou différentes.

10. Procédé (800) selon l'une des revendications 8 et 9, **caractérisé par** une étape (840) d'acheminement d'un flux de fluide jusque dans le boîtier (110) du dispositif de détection (100) et/ou à travers le boîtier (110) du dispositif de détection (100).
